# Europäisches Patentamt

## European Patent Office

## Office européen des brevets

(11) Publication number: **0 134 330**
**B1**

# (12) EUROPEAN PATENT SPECIFICATION

(45) Date of publication of patent specification: **30.09.87**

(51) Int. Cl.⁴: **B 01 J 29/28,** B 01 J 29/38, C 07 C 87/52

(21) Application number: **83304714.5**

(22) Date of filing: **15.08.83**

(54) **Treatment of zeolites.**

(43) Date of publication of application:
**20.03.85 Bulletin 85/12**

(45) Publication of the grant of the patent:
**30.09.87 Bulletin 87/40**

(84) Designated Contracting States:
**BE DE FR GB IT NL SE**

(56) References cited:
**EP-A-0 034 444**
**EP-A-0 062 542**
**BE-A- 764 917**
**US-A-3 835 028**

(73) Proprietor: **MOBIL OIL CORPORATION**
**150 East 42nd Street**
**New York New York 10017 (US)**

(72) Inventor: **Calvert, Robert Bruce**
**157 Hayward Mill Road**
**Concord, Mass. 01742 (US)**
Inventor: **Perkins, Patrick Danford**
**Wrick Avenue**
**Titusville New Jersey 08560 (US)**
Inventor: **Chang, Clarence Dayton**
**11 Murray Place**
**Princeton New Jersey (US)**

(74) Representative: **Grundy, Derek George Ritchie**
**et al**
**CARPMAELS & RANSFORD 43, Bloomsbury**
**Square**
**London WC1A 2RA (GB)**

Courier Press, Leamington Spa, England.

EP 0 134 330 B1

# 0 134 330

**Description**

This invention relates to treatment of zeolites to enhance their catalytic activity.

Zeolitic materials, both natural and synthetic, have been demonstrated in the past to have catalytic properties for various types of hydrocarbon conversions. Certain zeolitic materials are ordered, porous crystalline aluminosilicates having a definite crystalline structure containing a large number of channels. These cavities and channels are precisely uniform in size. Since the dimensions of these pores accept molecules of certain dimensions for adsorption while rejecting those of larger dimensions, these materials have come to be known as "molecular sieves" and are utilized in a variety of ways to take advantage of these properties. The present invention relates particularly to the relatively recently discovered class of such materials which is characterized by possessing a constraint index in the range 1 to 12. Constraint Index (C.I.) values for some typical materials are:

| Zeolite | C.I. |
| --- | --- |
| ZSM-4 | 0.5 |
| ZSM-5 | 8.3 |
| ZSM-11 | 8.7 |
| ZSM-12 | 2 |
| ZSM-23 | 9.1 |
| ZSM-35 | 4.5 |
| ZSM-38 | 2 |
| ZSM-48 | 3.4 |
| TMA Offretite | 3.7 |
| Clinoptilolite | 3.4 |
| H-Zeolon (mordenite) | 0.4 |
| REY | 0.4 |
| Amorphous silica-alumina | 0.6 |
| Erionite | 38 |

Zeolites ZSM-5, -11, -12, -23, -35, -38 and -48 are defined, respectively by the X-ray data set forth in US—A—3,702,886, 3,709,979, 3,832,449, 4,076,842, 4,016,245, 4,046,859 and EP—A—15132.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with other suitable metal cations of Groups I through VIII of the Periodic Table, including, by way of example, nickel, copper, zinc, palladium, calcium or rare earth metals.

In spite of the well-known attributes of the ZSM-5 family of zeolites, attempts have been made to improve these materials by further enhancing their hydrocarbon conversion activity. Such enhancement is desirable in order to improve the efficiency of catalysts in hydrocarbon conversion process and is of particular importance where shape selective catalysts are concerned. Shape selective catalysts are extremely useful in such processes as xylene isomerization, toluene disproportionation and p-ethyltoluene synthesis which require relatively high para-selectivity. Enhanced shape-selectivity of a catalyst composition can also improve the efficiency of methanol-to-gasoline (MTG) processes, methanol-to-olefin (MTO) processes, and catalytic dewaxing, as well as petro-chemical processes. Efforts to enhance the shape-selectivity of ZSM-5-type materials in sorptive and catalytic applications by calcining at temperatures of 600°C to 1300°C have generally met with the disappointing results. While such treated catalyst materials may exhibit enhanced shape-selectivity, they can suffer loss in catalytic activity. Exposure of zeolites to high temperatures results in a breakdown of crystalline structure and a corresponding loss in activity. Furthermore, zeolites which are calcined to an activity of about zero, generally prove extremely difficult, if not impossible, to reactivate.

There has now been discovered a process for enhancing the hydrocarbon conversion activities of zeolites such as ZSM-5, particularly the hydrogen forms, such as which have been substantially deactivated by calcining. Such catalysts are activated, according to the invention, by exposure to an activating agent comprising a mixture of steam and ammonia, at temperatures ranging from about 100 to 1000°C, preferably 400 to 600°C. Calcining and activation of the zeolite material is allowed to proceed to the extent that the resulting product retains a crystallinity of at least about 10%. The extent of crystallinity remaining within the product as it undergoes reactivation can be determined by conventional X-ray diffraction studies.

The activity of the resulting catalyst can be measured in terms of its alpha value. The alpha test is described in the Journal of Catalysis, Vol. VI, pages 278—287, 1966. The extent of the activation produced by the present method is notable. Significant increases in the alpha value may be obtained where ZSM-5 type zeolites are activated by the method of the present invention.

2

**0 134 330**

The activation process of the present invention may be used to produce a catalyst material of enhanced shape-selectivity having relatively high hydrocarbon conversion activity. The zeolites are calcined at temperatures ranging from about 600° to 1300°C, say about 1000°C in order to enhance their shape selectivity. The resulting materials, which suffer a loss of activity on calcination, are then activated by exposing the calcined compositions to a mixture of steam and ammonia at temperatures of about 100° to 1000°C. The calcining and activation steps are carried out to the extent that at least about 10% crystallinity is preserved in the resulting product. The molar ratio of steam to ammonia employed can range from about .01 to 10, preferably about 1.

The zeolite particles can be in the form of a powder, a granule, or a molded product, such as an extrudate having particle size sufficient to pass through a 2 mesh (Tyler) screen (50.8 mm) and be retained by a 400 mesh (Tyler) (0.037 mm) screen. In cases where the catalyst is molded, such as by extrusion, the catalyst crystals can be extruded before drying or dried or partially dried and then extruded.

The zeolite compositions resulting from the process of the invention can be used as catalysts in a number of important reactions. In particular, the subject shape-selectivity enhanced catalysts can be advantageously utilized in xylene isomerization, toluene disproportionation and p-ethyltoluene synthesis processes where high para-selectivity is desirable. The enhanced shape-selectivity of these compositions also commands their use in processes such as catalytic dewaxing. In addition these compositions are suitable for methanol conversion processes such as methanol-to-olefin, and methanol-to-gasoline where their use can result in lower durene (1,2,4,5-tetramethylbenzene) yields. The compositions of the present invention can also be used as supports for metals in dual-functional catalytic applications such as syngas reactions.

Alkylation of aromatic hydrocarbons, e.g., benzene, with an alkylating agent such as an alkyl halide, an alcohol or an olefin, can be readily effected in the presence of the treated composition as catalyst. Alkylation conditions include a temperature between about 204°C and about 538°C (about 400°F and about 1000°F) a pressure between about 25 and about 1000 p.s.i.g. (2.7 to 70 bar) utilizing an aromatic hydrocarbon/alkylating agent mole ratio of 2 to 200 and an alkylating agent weight hourly space velocity within the approximate range of 0.5 to 50.

Xylene isomerization is another reaction which can be suitably conducted in the presence of the described treated ZSM-5 composition as catalyst. Isomerization conditions include a temperature between about 149°C and about 482°C (about 300°F and about 900°F), a pressure between about 25 and about 1000 p.s.i.g. (2.7 to 70 bar), utilizing a weight hourly space velocity within the approximate range of 0.2 and 100.

Aromatics, such as, for example, toleune, may be disproportionated in the presence of the treated compositions at a temperature of from about 232°C to about 593°C (about 450°F to about 1100°F), a pressure of from about 50 p.s.i.g. to about 800 p.s.i.g. (4.5 to 56.2 bar) and a liquid hourly space velocity within the approximate range of about 0.1 to about 20. Aliphatic hydrocarbons may also be disproportionated in the presence of the described treated ZSM-5 compositions, at a temperature of from about 177°C to about 482°C (about 350°F to about 900°F) a pressure between 0 and 3000 p.s.i.g. (1 to 207.9 bar) and a liquid hourly space velocity of between about 0.01 and about 5.

Other reactions which can be accomplished employing the catalyst of this invention containing a metal, e.g., platinum, include hydrogenation-dehydrogenation reactions, desulfurization reactions, olefin polymerization (oligomerization) and other organic compound conversions, such as ammoniation of phenol wherein the deactivated catalyst is contacted with ammonia and water in the presence of phenol which activates the catalyst and produces aniline.

Examples 1—14
Effect of thermal treatments in ambient atmosphere on HZSM-5 zeolites

HZSM-5 samples having a silica to alumina ratio of about 70 were subjected to thermal treatments in ambient atmosphere under varying temperature conditions. Following these treatments, each sample was tested for n-hexane sorption and alpha value. Various samples were also tested for 3-methylpentane sorption, cyclohexane sorption, and crystallinity. HZSM-5 retained good crystallinity for periods in excess of one hour at temperatures below 1000°C. Above 1000°C the rate of framework collapse increased significantly. High temperature treated HZSM-5 samples were crystalline by X-ray and sorbed 7—12% n-hexane at 25°C after 20 minutes. The zeolites exhibited increased shape-selectivity as calcination temperatures increased. The HZSM-5 zeolites decreased cracking activity from $\alpha=150$ for parent materials to $\alpha=0$ for material calcined at 1000°C for one hour. Additional data relating to these thermal treatments can be found in Table I.

Example 15
Reactivation by contact with ammonia and steam

The catalyst of Example 5 was used to convert phenol to aniline by ammoniation. Phenol was reacted with excess $NH_3$ (0.5 WHSV) at 510°C, and 28 atm (28.4 bar). Steam was produced in situ during the reaction. Conversion of phenol was 94.4%. Selectivity to aniline was 90.2%. The catalyst became activated in situ by exposure to steam and ammonia, with the alpha increasing from 0.3 to 3.5. In a control run, ZSM-5 with $SiO_2/Al_2O_3$ 26,000 and alpha=0.3 converted only 1.5% phenol at the same conditions.

3

Example 16
Sorption and activity determination of reactivated catalyst

The reactivated catalyst of Example 10 was tested for sorption rates (percent by weight) and activity. n-Hexane sorption was 7.9%, cyclohexane sorption was "slow", toluene sorption was 1% and o-xylene sorption was less than .05%. n-Hexane and cyclohexane were sorbed for 20 minutes at 25°C while toluene and o-xylene sorption were measured at the end of 10 minutes sorption at 25°C. While sorption rates changed only slightly if at all upon treatment with ammonia and steam, the activity on reactivation changed from $\alpha=0$ to $\alpha=3.5$.

0 134 330

TABLE I
Effects of thermal treatments on ZSM-5 zeolites

| Example | Treatment | Atmosphere | n-Hexane sorption | 3-Methyl-pentane | Cyclo-hexane | α | X-ray crystallinity |
|---|---|---|---|---|---|---|---|
| | Parent HZSM-5 | ambient air | 11.6% | 8.9 | 8.7 | 192 | 100 |
| 1* | 1 hr @ 600°C | " | 11.6% | | | 113—175 | 90% |
| 2 | 1 hr @ 700°C | " | 11.2% | | | 35 | 90% |
| 3 | 1 hr @ 800°C | " | 10.7% | | | 12 (CI=1.4) | 82% |
| 4 | 1 hr @ 900°C | " | 9.8% | | | 4 (CI=0.4) | |
| 5 | 1 hr @ 1000°C | " | 8.3% | 5.4 | slow | 0 | 61% |
| 6 | 1 hr @ 1100°C | | | | | | 25% |
| 7 | 2 hrs @ 1100°C | " | 0 | | | 0 | 0% |
| 8 | 5 min. @ 1000°C | " | 10.2% | | | .1 | |
| 9 | 10 min. @ 1000°C | " | 10.0% | | | | |
| 10 | 15 min. @ 1000°C | " | 9.0% | | | | |
| 11 | 3.5 min. @ 1100°C | " | 7.5% | | | | |
| 12 | 10 min. @ 1100°C | " | 3.7% | | | | |
| 13 | 7.5 min. @ 1200°C | " | 4.1% | | | 0 | |
| 14 | 5 min. @ 1200°C | " | 0% | | | 0 | |

*note change w/time

# 0 134 330

Examples 17 to 19

Three HZSM-5 zeolite samples were calcined at 1000°C for one hour under ambient conditions. Their activities were measured as $a=1.2$. The samples were then treated at 400 p.s.i.g. (28.6 bar) and 510°C in a nitrogen atmosphere with, respectively, ammonia, steam, and a mixture of ammonia and steam. After such treatments the HZSM-5 samples were again measured for activity. The ammonia-treated catalyst was slightly increased in activity to $a=1.5$. The steam-treatment yielded a catalyst of decreased activity, $a=1$, while exposure to the ammonia/water mixture resulted in a significant increase in activity to $a=6$. Data for the three runs is given in Table III. The results of these runs show that activity of a calcined zeolite catalyst is significantly enhanced when contacted with a mixture of ammonia and steam.

TABLE III

Effects of exposure of calcined HZSM-5 to ammonia, steam and a mixture of ammonia and steam on catalyst activity

|  | Example 17 | Example 18 | Example 19 |
|---|---|---|---|
| Treatment mixture | $NH_3/N_2$ 10:1 mole ratio | $H_2O/N_2$ 2:1 mole ratio | $NH_3/H_2O/N_2$ 1.11:2.67:1.0 $(NH_3/H_2O)$ (0.45:1) |
| Temperature | 510°C | 510°C | 510°C |
| Pressure | 400 p.s.i.g. (28.6 bar) | 400 p.s.i.g. | 400 p.s.i.g. |
| Exposure time | 7.5 hours | 4 hours | 8 hours |
| $a$ | 1.5 | 1 | 6 |

## Claims

1. A method for activating a catalyst deactivated by calcination at a temperature of 1000 to 1300°C, said catalyst comprising a zeolite having a constraint index in the range 1 to 12, which comprises contacting said catalyst with an activating agent comprising ammonia and steam at a temperature from 100 to 1000°C to the extent that at least about 10% crystallinity is preserved in the activated product.

2. The method of claim 1 wherein the zeolite is ZSM-5, ZSM-11, ZSM-5/ZSM-11 intermediate, ZSM-12, ZSM-23, ZSM-35, ZSM-38 or ZSM-48.

3. The method of any preceding claim wherein the zeolite is in the hydrogen form.

4. The method of any preceding claim wherein the molar ratio of ammonia to steam ranges from about 0.01 to 10.

5. The method of claim 4 wherein the molar ratio of ammonia to steam is 0.75 to 1.25.

6. The method of any preceding claim wherein the zeolite is contacted with said activating agent at a temperature of 400 to 600°C.

7. The method of claim 1 wherein the deactivated catalyst, in the presence of phenol, is contacted with ammonia and water which activates the catalyst and produces aniline.

## Patentansprüche

1. Verfahren zur Aktivierung eines deaktivierten Katalysators durch Kalzinierung bei einer Temperatur von 1000 bis 1300°C, wobei der Katalysator einen Zeolith mit einem Zwangsindex im Bereich von 1 bis 12 umfaßt, bei dem der Katalysator mit einem Aktivierungsmittel, das Ammoniak und Dampf umfaßt, bei einer Temperatur von 100 bis 1000°C in einem solchen Ausmaß in Kontakt gebracht wird, daß etwa 10% der Kristallinität in dem aktivierten Produkt erhalten bleiben.

2. Verfahren nach Anspruch 1, worin der Zeolith ZSM-5, ZSM-11, ZSM-5/ZSM-11-Zwischenprodukt, ZSM-12, ZSM-23, ZSM-35, ZSM-38 oder ZSM-48 ist.

3. Verfahren nach einem der vorstehenden Ansprüche, worin der Zeolith in der Wasserstoffform vorliegt.

4. Verfahren nach einem der vorstehenden Ansprüche, worin das Molverhältnis von Ammoniak zu Dampf im Bereich von etwa 0,01 bis 10 liegt.

5. Verfahren nach Anspruch 4, worin das Molverhältnis von Ammoniak zu Dampf, 0,75 bis 1,25 beträgt.

6. Verfahren nach einem der vorstehenden Ansprüche, worin der Zeolith mit dem Aktivierungsmittel bei einer Temperatur von 400 bis 600°C in Kontakt gebracht wird.

**0 134 330**

7. Verfahren nach Anspruch 1, worin der deaktivierte Katalysator in Gegenwart von Phenol mit Ammoniak und Wasser in Kontakt gebracht wird, das den Katalysator aktiviert und Anilin erzeugt.

**Revendications**

1. Un procédé pour activer un catalyseur désactivé par calcination, à une température de 1 000°C à 1 300°C, ce catalyseur comprenant une zéolite présentant un indice de contrainte compris entre 1 et 12, qui consiste à mettre ledit catalyseur au contact d'un agent d'alkylation comprenant de l'ammoniac et de la vapeur, à une température comprise entre 100°C et 1 000°C, de façon à ce qu'au moins environ 10% de cristallinité soient préservés dans le produit activé.

2. Le procédé selon la revendication 1, dans lequel la zéolite consiste en ZSM-5, ZSM-11, un intermédiaire de ZSM-5/ZSM-11, ZSM-12, ZSM-23, ZSM-35, ZSM-38 et ZSM-48.

3. Le procédé selon l'une quelconque des revendications précédentes, dans lequel la zéolite est sous forme protonée.

4. Le procédé selon l'une quelconque des revendications précédentes, dans lequel le rapport molaire ammoniac/vapeur est compris entre 0,01 et 10 environ.

5. Le procédé selon la revendication 4, dans lequel le rapport molaire ammoniac/vapeur est compris entre 0,75 et 1,25.

6. Le procédé selon l'une quelconque des revendications précédentes, dans lequel la zéolite est mise au contact dudit agent d'activation à une température comprise entre 400°C et 600°C.

7. Le procédé selon la revendication 1, dans lequel le catalyseur désactivé, en présence de phénol, est mis au contact d'ammoniac et d'eau qui activent le catalyseur et produisent de l'aniline.